# EUROPEAN PATENT APPLICATION

(11) **EP 4 361 623 A1**
(43) Date of publication of application: **01.05.2024**
(21) Application number: 23206039.2
(22) Date of filing: 26.10.2023
(51) Int. Cl.: G01N 27/447

(54) **SAMPLE ANALYSIS METHOD, CAPILLARY ELECTROPHORESIS SOLUTION, AND SAMPLE ANALYSIS KIT**

(30) Priority: 28.10.2022 JP 2022173451
(71) Applicant: ARKRAY, Inc., Kyoto-shi, Kyoto 601-8045 (JP)
(72) Inventor: OGA, Misaki, Kyoto, 602-0008 (JP); ONUMA, Naotsugu, Kyoto, 602-0008 (JP)
(74) Representative: Dehns

(57) **Abstract**

Provided are a sample analysis method including separating albumin and γ-globulin from a sample containing albumin and γ-globulin, in an alkaline solution by capillary electrophoresis, in which the alkaline solution contains a cationic polymer, and the like.

## Description

### BACKGROUND

### Technical Field

The present disclosure relates to a sample analysis method, a capillary electrophoresis solution, and a sample analysis kit.

### Related Art

In the field of clinical examination, proteins such as hemoglobin contained in erythrocytes, albumin, globulin or the like contained in serum, and the like are routinely analyzed.

For example, in hemoglobin, there is HbA1c as an analysis target for diagnosing diabetes or grasping a medical condition. HbS (sickle-cell hemoglobin), HbC, HbD, HbE, HbA2, HbF, and the like are widely used as analysis targets for diagnosing abnormal hemoglobinopathy and β-thalassemia symptoms. Japanese Patent Application Laid-Open (JP-A) No. 2016-136135 discloses a sample analysis method including separating various kinds of hemoglobin in an alkaline solution containing a cationic polymer by capillary electrophoresis.

Albumin and γ-globulin in the serum are used as indices of various diseases such as multiple myeloma, nephrotic syndrome, liver cirrhosis, and nutritional disorder.

For example, for a patient with multiple myeloma, normal antibody production is not performed due to canceration of plasma cells in the bone marrow, and an abnormally produced monoclonal immunoglobulin or a part thereof exhibits M-proteinemia that appears in the serum or urine. Therefore, M protein is an important index that serves as a guide for the diagnosis and therapeutic effect of multiple myeloma. In the clinical examination, as a method of measuring albumin and γ-globulin, a BCP improvement method, a Biuret method, a serum protein fractionation method using an electrophoresis method, and the like are known, but measurement by an electrophoresis method is essential for detection of M protein.

Usually, most of immunoglobulins in serum are polyclonal antibodies, and thus show a broad peak in the γ-globulin fraction in a case in which electrophoresis is performed.

On the other hand, M protein is a monoclonal antibody and has a single charge, and thus shows a sharp peak called M peak in the γ-globulin fraction. Therefore, in the electrophoresis method, the presence or absence of M protein can be distinguished.

As described above, the examination of serum proteins such as albumin and γ-globulin by an electrophoresis method is useful for detecting M protein and grasping the clinical conditions of various diseases, and thus is widely used as a screening examination for serum protein abnormalities.

Among protein fraction examinations using an electrophoresis method, a capillary electrophoresis method has been increasingly demanded in recent years since it has a short analysis time and can process many specimens.

As a method of separating albumin and γ-globulin by a capillary electrophoresis method, for example, JP-A No. 2004-517338 discloses an alkaline pH, free solution capillary electrophoresis method for analyzing a clinical sample containing protein constituents, the method including at least one step of introducing the sample into a capillary tube containing a buffer system, which contains a biological buffer solution with a pKa in a range of from 8.8 to 10.7 at 25°C and at least one additive capable of increasing an ionic strength of the buffer system, and discloses that each globulin containing albumin and γ-globulin contained in serum having monoclonal gammopathy is separated within 10 minutes.

Japanese National-Phase Publication (JP-A) No. 2004-517339 discloses an alkaline pH, free solution capillary electrophoresis method for analyzing a sample containing a protein constituent, the method including at least one step of introducing the sample into a capillary tube including a buffer system, the buffer system further containing at least one additive capable of hydrophobically interacting with one or more protein constituents and capable of providing the protein constituent or constituents with one or more negative charges and of modifying the electrophoretic mobility, in which each globulin containing albumin and γ-globulin contained in normal serum is separated within 10 minutes.

### SUMMARY

In the conventional capillary electrophoresis methods, it takes about 10 minutes to separate albumin and γ-globulin, and in the case of analyzing a large amount of specimen in the field of clinical examination or the like, it is necessary to prepare a system including a plurality of capillaries in order to shorten the separation time.

An object of an embodiment of the disclosure is to provide a sample analysis method capable of separating albumin and γ-globulin from a sample containing albumin and γ-globulin, in a shorter time than in a conventional method, a capillary electrophoresis solution, and a sample analysis kit.

A sample analysis method according to an embodiment of the disclosure includes: a separation step of separating albumin and γ-globulin from a sample containing albumin and γ-globulin, in an alkaline solution by capillary electrophoresis,
in which the alkaline solution contains a cationic polymer.

According to an embodiment of the disclosure, it is possible to provide a sample analysis method capable of separating albumin and γ-globulin from a sample containing albumin and γ-globulin, in a shorter time than in a conventional method, a capillary electrophoresis solution, and a sample analysis kit.

### BRIEF DESCRIPTION OF THE DRAWINGS

Exemplary embodiments of the present disclosure will be described in detail based on the following figures, wherein:
Fig. 1A is a top view illustrating an embodiment of a capillary electrophoresis chip. Fig. 1B is a cross-sectional view of the electrophoresis chip illustrated in Fig. 1A.
Fig. 2A shows an electropherogram obtained using Sample A in a sample analysis method of Example 1. Fig. 2B shows an electropherogram obtained using Sample B in a sample analysis method of Example 1.
Fig. 3A shows an electropherogram obtained using Sample A in a sample analysis method of Example 2. Fig. 3B shows an electropherogram obtained using Sample B in a sample analysis method of Example 2.
Fig. 4 shows an electropherogram obtained using Sample B in a sample analysis method of Example 3.
Fig. 5A shows an electropherogram obtained using Sample C in a sample analysis method of Example 4. Fig. 5B shows an electropherogram obtained using Sample D in a sample analysis method of Example 4.
Fig. 6 shows an electropherogram obtained using Sample E in a sample analysis method of Example 5.
Fig. 7 shows an electropherogram obtained using Sample F in a sample analysis method of Example 6.
Fig. 8 shows an electropherogram obtained using Sample G in a sample analysis method of Comparative Example 1.
Fig. 9 shows an electropherogram obtained using Sample H in a sample analysis method of Comparative Example 2.

### DETAILED DESCRIPTION

An exemplary embodiment of the present invention will be described. These descriptions and examples illustrate embodiments and do not limit the scope of the invention.

In the present specification, "to" indicating a numerical range is used to mean that numerical values which are described before and after "to" are included as a lower limit value and an upper limit value, respectively.

In a numerical range described in a stepwise manner in the disclosure, an upper limit value or a lower limit value described in one numerical range may be replaced with an upper limit value or a lower limit value described in another numerical range described in a stepwise manner. In a numerical range described in the disclosure, an upper limit value or a lower limit value of the numerical range may be replaced with a value shown in Examples.

Each component may contain a plurality of corresponding substances.

When the amount of each component in a composition is referred to, in a case in which a plurality of substances corresponding to each component are present in the composition, the amount of each component in the composition means the total amount of the plurality of substances present in the composition unless otherwise specified.

In the disclosure, the term "step" includes not only an independent step but also a step by which an intended action of the step is achieved, though the step cannot be clearly distinguished from other steps.

In the disclosure, a combination of two or more preferred aspects is a more preferred aspect.

In a case in which an embodiment is described with reference to the drawings in the disclosure, the configuration of the embodiment is not limited to the configuration illustrated in the drawings. The sizes of members in each drawing are conceptual, and the relative relationship between the sizes of the members is not limited thereto.

### [Sample Analysis Method]

A sample analysis method according to an embodiment of the disclosure (hereinafter, also referred to as "specific sample analysis method") includes a separation step of separating albumin and γ-globulin from a sample containing albumin and γ-globulin, in an alkaline solution by capillary electrophoresis, in which the alkaline solution (hereinafter, also referred to as "specific alkaline solution") contains a cationic polymer.

According to the specific sample analysis method, albumin and γ-globulin can be separated from a sample containing albumin and γ-globulin, in a shorter time than in a conventional method. The reason why the above effect is exhibited is not clear, but it is presumed as follows.

Albumin and γ-globulin, which are substances to be analyzed, are negatively charged in an alkaline solution. In a case in which a negative electrode is brought into contact with the sample introduction side and a voltage is applied to the substance to be analyzed, these substances to be analyzed move to the positive electrode side due to the charge of the substance to be analyzed and the electroosmotic flow.

On the other hand, since the cationic polymer has a cationic group, the cationic polymer moves from the positive electrode side to the negative electrode side. During electrophoresis, the substance to be analyzed and the cationic polymer repeatedly interact with each other via bonding and dissociation in the capillary, whereby the substance to be analyzed is positively charged, so that a force for pulling back the substance to be analyzed to the negative electrode side is generated. Since the cationic polymer has a large charge, a charge difference between the substances to be analyzed, which have interacted with the cationic polymer, is larger than a charge difference inherent in the substance to be analyzed. Therefore, it is presumed that albumin and γ-globulin can be efficiently separated in a short time since a difference in electrophoretic velocity between the substances to be analyzed increases.

### <Separation Step>

The specific sample analysis method includes a separation step of separating albumin and γ-globulin from a sample containing albumin and γ-globulin, in a specific alkaline solution by capillary electrophoresis.

Separation of albumin and γ-globulin in the sample, in the specific alkaline solution by capillary electrophoresis can be performed by introducing the sample into a capillary flow path into which the specific alkaline solution is filled, and applying a voltage to the whole or a part of the capillary flow path after introduction of the sample.

By applying the voltage, albumin and γ-globulin in the sample can be electrophoresed and separated.

The voltage can be applied to the capillary flow path by bringing a negative electrode into contact with the sample introduction side of the capillary flow path and bringing a positive electrode into contact with the specific alkaline solution supply side.

The cross-sectional shape of the capillary flow path is not particularly limited, and may be a circular shape, a rectangular shape, or other shapes.

In the case of a rectangular shape, each of the flow path height and the flow path width of the capillary flow path is preferably from 1 µm to 1000 µm, more preferably from 10 µm to 200 µm, and still more preferably from 25 µm to 100 µm. In the case of a circular shape, the inner diameter of the capillary flow path is preferably 10 µm or more or 25 µm or more and is preferably 100 µm or less or 75 µm or less.

The flow path length of the capillary flow path is preferably from 10 mm to 150 mm and more preferably from 20 mm to 60 mm.

Examples of the material for the capillary flow path include glass, fused silica, and plastic. Examples of the plastic include polymethyl methacrylate (PMMA), polycarbonate, polystyrene, polytetrafluoroethylene (PTFE), and polyetheretherketone (PEEK).

The separation step may be performed using a capillary electrophoresis chip in which the capillary flow path is made into a microchip.

The capillary electrophoresis chip can include a sample holding tank, an electrophoretic liquid holding tank, and a capillary flow path, in which the sample holding tank and the electrophoretic liquid holding tank are communicated with each other via the capillary flow path.

The size of the capillary electrophoresis chip is not particularly limited, and is preferably appropriately adjusted. The size of the capillary electrophoresis chip can be, for example, from 10 mm to 200 mm in length, from 1 mm to 60 mm in width, and from 0.3 mm to 5 mm in thickness.

The volume of each of the sample holding tank and the electrophoretic liquid holding tank is appropriately determined according to the inner diameter and the length of the capillary flow path, and is preferably from 1 mm³ to 1000 mm³ and more preferably from 5 mm³ to 100 mm³.

The amount of the sample to be filled into the sample holding tank is not particularly limited, and can be from 1 µL to 70 µL.

The amount of the specific alkaline solution to be filled into the electrophoretic liquid holding tank is not particularly limited, and can be from 1 µL to 70 µL.

The voltage applied to both ends of the capillary flow path is preferably from 500 V to 10000 V, and more preferably from 500 V to 5000 V

The electrophoresis time of capillary electrophoresis in the separation step is preferably from 50 seconds to less than 250 seconds, and more preferably from 60 seconds to 200 seconds.

In the capillary flow path, a liquid flow from the negative electrode side toward the positive electrode side may be generated. Examples of the liquid flow include an electroosmotic flow.

The inner wall of the capillary flow path is preferably coated with a cationic substance or an anionic substance.

By coating the inner wall of the capillary flow path with a cationic substance, the inner wall of the capillary flow path can be positively charged. As a result, an electroosmotic flow from the negative electrode side toward the positive electrode side can be easily generated in the capillary flow path.

In a case in which the inner wall of the capillary flow path is coated with the anionic substance, the inner wall of the capillary flow path is negatively charged, but the cationic polymer contained in the specific alkaline solution is bonded to the inner wall of the negatively charged capillary flow path. As a result, the inner wall of the capillary flow path is positively charged, and the electroosmotic flow from the negative electrode side toward the positive electrode side can be easily generated in the capillary flow path as described above.

The cationic substance is not particularly limited, and a silane coupling agent having a cationic functional group and the like can be used. From the viewpoint of improving the separation accuracy, the cationic substance is preferably a polymer having a quaternary ammonium base.

The anionic substance is not particularly limited, and polysaccharides having an anionic group, a silane coupling agent having an anionic functional group, and the like can be used.

Examples of the polysaccharides having an anionic group include sulfated polysaccharides, carboxylated polysaccharides, sulfonated polysaccharides, and phosphorylated polysaccharides.

Examples of the sulfated polysaccharides include chondroitin sulfate, heparin, heparan, fucoidan, and salts thereof. Examples of the carboxylated polysaccharides include alginic acid, hyaluronic acid, and salts thereof.

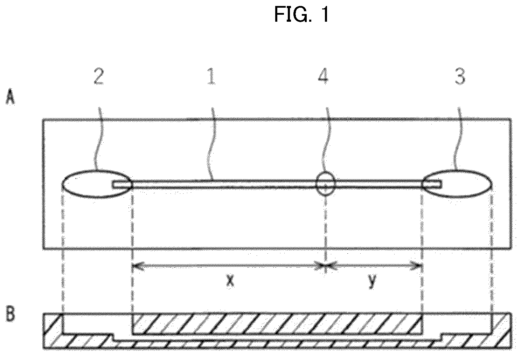

Figs. 1A and 1B illustrate an embodiment of a capillary electrophoresis chip. Fig. 1A is a top view illustrating an embodiment of a capillary electrophoresis chip, and Fig. 1B is a cross-sectional view of the electrophoresis chip illustrated in Fig. 1A.

The capillary electrophoresis chip illustrated in Figs. 1A and 1B includes a capillary flow path 1, a sample holding tank 2, and an electrophoretic liquid holding tank 3, in which the sample holding tank 2 and the electrophoretic liquid holding tank 3 are communicated with each other via the capillary flow path 1. The capillary electrophoresis chip has a detection unit 4 for detecting albumin and γ-globulin that have been separated from a sample in the capillary flow path 1. The detection unit 4 may be arranged around a periphery of the capillary flow path 1 (for example, below the capillary flow path 1).

Each of the sample holding tank 2 and the electrophoretic liquid holding tank 3 may includes an electrode for applying a voltage to both ends of the capillary flow path 1 (not illustrated). Specifically, the sample holding tank 2 (sample introduction side) can include a negative electrode, and the electrophoretic liquid holding tank 3 (specific alkaline solution supply side) can include a positive electrode.

The position of the detection unit 4, that is, the length required for separation (distance from the sample holding tank 2 to the detection unit 4, x in Fig. 1A) can be appropriately determined according to the length of the capillary flow path 1 or the like. In a case in which the length (x + y in Fig. 1A) of the capillary flow path 1 is from 10 mm to 150 mm, the distance (x) from the sample holding tank 2 to the detection unit 4 is preferably from 5 mm to 140 mm, more preferably from 10 mm to 100 mm, and still more preferably from 15 mm to 50 mm.

### -Specific Alkaline Solution-

In the disclosure, "alkaline" means that the pH is more than 7.0. The pH of the specific alkaline solution is preferably higher than the isoelectric points of albumin and γ-globulin, and is preferably from 8.5 to 12.0, more preferably from 9.0 to 11.0, and still more preferably from 9.0 to 10.0.

In the disclosure, the pH of the specific alkaline solution is the pH of the specific alkaline solution at 25°C, and is measured using a pH meter 30 minutes after the electrode is immersed. As the pH meter, F-72 manufactured by HORIBA, Ltd. or a device similar thereto can be used.

The specific alkaline solution contains a cationic polymer. The specific alkaline solution may contain two or more cationic polymers.

In the disclosure, the "cationic polymer" means a polymer having a cationic group.

In the disclosure, the "cationic group" includes a cationic group and a group that is ionized to become a cationic group.

Examples of the cationic group include a primary amino group, a secondary amino group, a tertiary amino group, a quaternary ammonium base, and an imino group. Among them, from the viewpoint of shortening the separation time of albumin and γ-globulin, a primary amino group or a secondary amino group is preferable, and a secondary amino group is more preferable.

The cationic polymer is preferably water-soluble. In the disclosure, "water-soluble" means that a target substance is dissolved in an amount of 1 mass% or more in water at 25°C.

Examples of the cationic polymer having primary to tertiary amino groups or a cationic group that can be ionized into primary to tertiary amino groups include polyallylamine, polyvinylamine, polylysine, polyarginine, polyhistidine, polyornithine, polydiallylamine, polymethyldiallylamine, polyethyleneimine, diallylamine-acrylamide polymer, dimethylamine-ammonia-epichlorohydrin polymer, and allylamine-diallylamine polymer.

Examples of the cationic polymer having an imino group or a cationic group that can be ionized into an imino group include polyethyleneimine.

Examples of the cationic polymer having a quaternary ammonium base or a cationic group that can be ionized into a quaternary ammonium base include polyquaternium, dimethylamine-ammonia-epichlorohydrin polymer, dimethylamine-epichlorohydrin polymer, and diallyldimethylammonium chloride polymer.

In the disclosure, "polyquaternium" refers to a cationic polymer including a constituent unit derived from a monomer having a quaternary ammonium group. Polyquaternium can be confirmed by INCI (International Nomenclature for Cosmetic Ingredients) directory. In one or more embodiments, examples of the polyquaternium include polydiallyl dimethyl ammonium salts such as polyquaternium-6 (poly(diallyl dimethyl ammonium chloride), polyquaternium-7 (copolymer of acrylamide and diallyl dimethyl ammonium chloride), polyquaternium-4 (diallyl dimethyl ammonium chloride-hydroxyethyl cellulose copolymer), and polyquaternium-22 (copolymer of acrylic acid and diallyl dimethyl ammonium chloride), and polyquaternium-2 (poly[bis(2-chloroethyl)ether-alt-1,3-bis[3-(dimethylamino)propyl]urea]).

As the cationic polymer, in addition to the ammonium salt, in one or more embodiments, a cationic polymer of an onium salt such as a phosphonium salt, an oxonium salt, a sulfonium salt, a fluoronium salt, or a chloronium salt can also be used.

Examples of the cationic polymer having a hydrazide group or a cationic group that can be ionized into a hydrazide group include aminopolyacrylamide.

From the viewpoint of shortening the separation time of albumin and γ-globulin, the cationic polymer preferably includes at least one selected from the group consisting of dimethylamine-ammonia-epichlorohydrin polymer, dimethylamine-epichlorohydrin polymer, diallylamine-acrylamide polymer, polyethyleneimine, polyallylamine, diallylamine polymer, allylamine-diallylamine polymer, methyldiallylamine polymer, and diallyldimethylammonium chloride polymer, more preferably includes at least one selected from the group consisting of dimethylamine-ammonia-epichlorohydrin polymer, dimethylamine-epichlorohydrin polymer, diallylamine-acrylamide polymer, polyethyleneimine, and polyallylamine, and still more preferably includes at least one selected from the group consisting of dimethylamine-ammonia-epichlorohydrin polymer and diallylamine-acrylamide polymer.

The cationic polymer described above may be in the form of a salt, and examples thereof include hydrochloride.

From the viewpoint of shortening the separation time of albumin and γ-globulin, the weight average molecular weight of the cationic polymer is preferably from 10,000 to 500,000, more preferably from 12,000 to 300,000, still more preferably from 15,000 to 150,000, particularly preferably from 20,000 to 130,000, and most preferably from 20,000 to 100,000.

The reason why the separation time of albumin and γ-globulin is shortened by setting the weight average molecular weight of the cationic polymer within the above numerical range is not clear, but it is presumed as follows.

The isoelectric points of albumin and γ-globulin (IgG as a representative component) are around pH 5 and around pH 5 to 9, respectively.

Since albumin has a lower isoelectric point than γ-globulin, the negative charge of albumin is larger than that of γ-globulin in the alkaline solution, so that the interaction with the cationic polymer is strong.

Therefore, it is presumed that by setting the weight average molecular weight of the cationic polymer to 500,000 or less, it is possible to suppress an excessive increase in difference between the force for pulling back albumin to the negative electrode side and the force for pulling back γ-globulin to the negative electrode side, which are generated by the interaction with the cationic polymer, and it is possible to further shorten the separation time of albumin and γ-globulin.

It is presumed that by setting the weight average molecular weight of the cationic polymer to 10,000 or more, it is possible to suppress an excessive decrease in difference between the force for pulling back albumin to the negative electrode side and the force for pulling back γ-globulin to the negative electrode side, which are generated by the interaction with the cationic polymer, and the separation accuracy can be improved.

In the disclosure, the weight average molecular weight of the cationic polymer refers to the catalogue value. In a case in which there is no catalog value of the weight average molecular weight, the weight average molecular weight is a weight average molecular weight in terms of polyethylene glycol measured by a gel permeation chromatography (GPC) method.

From the viewpoint of shortening the separation time of albumin and γ-globulin, the content ratio of the cationic polymer with respect to the total mass of the specific alkaline solution is preferably from 0.01 mass% to 10 mass%, more preferably from 0.05 mass% to 8.0 mass%, still more preferably from 0.1 mass% to 5.0 mass%, and still more preferably from 1.0 mass% to 3.0 mass%.

As the cationic polymer, one synthesized by a conventionally known method may be used, or a commercially available one may be used.

The specific alkaline solution may contain water. Examples of water include distilled water, ion-exchanged water, pure water, and ultrapure water.

The content ratio of water with respect to the total mass of the specific alkaline solution is not particularly limited, and can be from 10 mass% to 99.9 mass%.

The specific alkaline solution may contain additives such as a non-surfactant-type zwitterionic substance (non-surfactant-type betaine or the like), a pH buffering substance, and a preservative for suppressing proliferation of microorganisms, and the like. Examples of the preservative include sodium azide, ethylparaben, and Proclin.

In the disclosure, the "non-surfactant-type zwitterionic substance" means a zwitterionic substance that does not form micelles. In the disclosure, "not forming micelles" means not forming micelles in an aqueous medium or not substantially forming micelles. In the disclosure, "not substantially forming micelles" means that the critical micelle concentration is preferably 200 mmol/L or more and more preferably 300 mmol/L or more, and still more preferably, a zwitterionic substance has not critical micelle concentration.

In the disclosure, "zwitterionic substance" means a compound having a positively charged group and a negatively charged group at positions not adjacent to each other in the same molecule, having no hydrogen atom capable of being dissociated bonded to an atom having a positive charge, and having no charge as a whole molecule.

### -Sample-

The sample contains albumin and γ-globulin.

Examples of γ-globulin include IgG, IgM, IgA, IgD, and IgE, and the sample may contain at least one selected from the group consisting of IgG, IgM, IgA, IgD, and IgE, and may contain two or more selected from the group consisting of IgG, IgM, IgA, IgD, and IgE.

The contents of albumin and γ-globulin with respect to the total mass of the sample are particularly limited, and can be set from 0.001 mass% to 100 mass%.

The form of the sample is not particularly limited, and may be those prepared from a sample raw material or may be a sample raw material itself.

Examples of the sample raw material include raw materials containing albumin and γ-globulin, and biological samples.

Examples of the biological sample include blood, serum, plasma, and those prepared from these, serum and plasma are preferable, and serum is more preferable.

Examples of blood, serum, and plasma include blood, serum, and plasma collected from a living body, and include blood, serum, and plasma of mammals other than human, and blood, serum, and plasma of human.

From the viewpoint of improving the separation accuracy, the sample preferably contains an alkaline solution containing a cationic polymer.

The sample containing the alkaline solution can be obtained by diluting the sample raw material using the alkaline solution. The dilution rate is preferably from 1.2 times to 100 times, more preferably from 2 times to 60 times, and still more preferably from 3 times to 50 times on a volume basis. The material used for dilution is not particularly limited, and examples thereof include a pH adjusting agent (for example, hydrochloric acid or the like), a surfactant (for example, EMULGEN LS-110 (manufactured by Kao Corporation) or the like), an antiseptic (for example, sodium azide or the like), an ionic strength adjusting agent (for example, sodium chloride or the like), and a refractive index adjusting agent (for example, saccharides such as sucrose).

The alkaline solution may be the same as or different from the specific alkaline solution filled into the capillary flow path.

### <Detection Step>

The specific sample analysis method can include a detection step of detecting albumin and γ-globulin separated in the separation step.

Albumin and γ-globulin can be detected by an optical method. Examples of the detection by an optical method include measurement of absorbance.

More specifically, albumin and γ-globulin can be detected by irradiating the separated albumin and γ-globulin with light having a wavelength of 280 nm to obtain an absorbance spectrum in which the vertical axis represents absorbance and the horizontal axis represents time.

In the case of using a capillary electrophoresis chip for separating albumin and γ-globulin, it is preferable to irradiate the detection unit with light having a wavelength of 280 nm.

Albumin and γ-globulin may be detected using an electropherogram (differential waveform) obtained by differentiating the waveform of the absorbance spectrum with respect to time.

An embodiment of the specific sample analysis method will be described with reference to Figs. 1A and 1B. The specific sample analysis method is not limited to that described below.

First, the specific alkaline solution containing a cationic polymer is filled as an electrophoretic liquid into the electrophoretic liquid holding tank 3 of the capillary electrophoresis chip, and the specific alkaline solution is filled into the capillary flow path 1 by capillary action.

Next, the sample is added to the sample holding tank 2 of the capillary electrophoresis chip into which the specific alkaline solution is filled.

The sample to be added to the sample holding tank 2 can be prepared by diluting serum, which is a sample raw material, with the alkaline solution.

A negative electrode is brought into contact with the sample holding tank 2 and a positive electrode is brought into contact with the electrophoretic liquid holding tank 3 (not illustrated), and a voltage is applied to both ends of the capillary flow path 1, that is, between the sample holding tank 2 and the electrophoretic liquid holding tank 3. As a result, the sample is introduced from the sample holding tank 2 into the capillary flow path 1, the sample containing albumin and γ-globulin moves from the sample holding tank 2 toward the electrophoretic liquid holding tank 3, and albumin and γ-globulin are separated.

In the detection unit 4, albumin and γ-globulin are detected by irradiation with light having a wavelength of 280 nm and measurement of absorbance by an absorbance measuring device.

The sample analysis method of the disclosure can be used for diagnosis, treatment, and the like of multiple myeloma, nephrotic syndrome, liver cirrhosis, nutritional disorder, and the like.

### [Capillary Electrophoresis Solution]

A capillary electrophoresis solution according to an embodiment of the disclosure contains a cationic polymer, the capillary electrophoresis solution being used for separation of albumin and γ-globulin by capillary electrophoresis.

A preferred embodiment of the capillary electrophoresis solution is the same as the specific alkaline solution used in the specific sample analysis method, and thus the description thereof is omitted here.

### [Sample Analysis Kit]

A sample analysis kit according to an embodiment of the disclosure includes a container containing the above-described capillary electrophoresis solution, and an electrophoresis chip including a sample holding tank, an electrophoretic liquid holding tank, and a capillary flow path, in which the sample holding tank and the electrophoretic liquid holding tank are communicated with each other via the capillary flow path.

Preferred embodiments of the capillary electrophoresis solution and the electrophoresis chip are the same as those of the specific alkaline solution and the electrophoresis chip used in the specific sample analysis method, and thus the description thereof is omitted here.

Examples of the material for the container containing the capillary electrophoresis solution include glass, fused silica, and plastic. The plastic has been described above, and thus the description thereof is omitted here.

The disclosure may relate to the following embodiments.
<1> A sample analysis method, including a separation step of separating albumin and γ-globulin from a sample comprising albumin and γ-globulin, in an alkaline solution by capillary electrophoresis,
   in which the alkaline solution contains a cationic polymer.
<2> The sample analysis method according to <1>, wherein a weight average molecular weight of the cationic polymer is from 10,000 to 500,000.
<3> The sample analysis method according to <1> or <2>, wherein a weight average molecular weight of the cationic polymer is from 15,000 to 150,000.
<4> The sample analysis method according to any one of <1> to <3>, wherein the cationic polymer includes at least one selected from the group consisting of dimethylamine-ammonia-epichlorohydrin polymer, dimethylamine-epichlorohydrin polymer, diallylamine-acrylamide polymer, polyethyleneimine, polyallylamine, diallylamine polymer, allylamine-diallylamine polymer, methyldiallylamine polymer, and diallyldimethylammonium chloride polymer.
<5> The sample analysis method according to any one of <1> to <4>, wherein a content ratio of the cationic polymer with respect to a total mass of the alkaline solution is from 0.01 mass% to 10 mass%.
<6> The sample analysis method according to any one of <1> to <5>, wherein an electrophoresis time of capillary electrophoresis in the separation is from 50 seconds to less than 250 seconds.
<7> The sample analysis method according to any one of <1> to <6>, wherein a pH of the alkaline solution is from 8.5 to 12.0.
<8> A capillary electrophoresis solution, containing a cationic polymer, in which the capillary electrophoresis solution is used for separation of albumin and γ-globulin by capillary electrophoresis.
<9> A sample analysis kit, including:
   a container containing the capillary electrophoresis solution according to <8>; and
   an electrophoresis chip including a sample holding tank, an electrophoretic liquid holding tank, and a capillary flow path,
   in which the sample holding tank and the electrophoretic liquid holding tank are communicated with each other via the capillary flow path.

### EXAMPLES

Examples will be described below, but the disclosure is not limited to these Examples at all. In the following description, unless otherwise specified, "part(s)" and "%" are all on a mass basis.

In the following Examples and Comparative Examples, electrophoresis was performed by a continuous sample introduction method.

### <Separation Device and Measuring Apparatus>

As a separation device, a resin chip having the capillary flow path 1 with the structure illustrated in Fig. 1 (flow path width: 40 µm, flow path height: 40 µm, flow path length: 30 mm, distance (x) from the sample holding tank 2 to the detection unit 4: 20 mm) was used. The capacity of each of the sample holding tank 2 and the electrophoretic liquid holding tank 3 was set to 60 µL. The inner wall of the capillary flow path was coated with polydiallyldimethylammonium chloride.

As a measuring device, an electrophoresis device self-manufactured was used.

### «Example 1»

The following substances were mixed, and sodium hydroxide and water were added until the pH reached 9.8 to prepare a specific alkaline solution (capillary electrophoresis solution) 1. The content ratio of the cationic polymer with respect to the total mass of the specific alkaline solution 1 was set to 1.5 mass%.

### (Composition of Specific Alkaline Solution 1)

· Dimethylamine-ammonia-epichlorohydrin polymer 1 (cationic polymer, KHE102L manufactured by SENKA corporation, weight average molecular weight: from 20,000 to 100,000) 1.5 mass%
· Sodium azide 0.02 mass%
· EMULGEN LS-110 (manufactured by Kao Corporation) 0.1 mass%
· Sodium hydroxide aqueous solution
· Water

An albumin/y-globulin mixed solution 1 having the following composition was prepared.

### (Composition of Albumin/y-Globulin Mixed Solution 1)

· 250 mg/mL albumin (manufactured by Wako, 010-27601) 25 mass/vol%
· 250 mg/mL γ-globulin (manufactured by Sigma, G4386-1G) 25 mass/vol%

The albumin/y-globulin mixed solution 1 was diluted (dilution rate 41 times (volume basis)) with a diluent 1 (pH 8.8) having the following composition to obtain Sample A.

In the diluent 1, hydrochloric acid and water were added until the pH of the solution reached 8.8.

### (Composition of Diluent 1)

· Polyethyleneimine (cationic polymer, manufactured by Wako, weight average molecular weight: 70,000) 0.75 mass%
· Sucrose 7.87 mass%
· Sodium chloride 0.26 mass%
· Sodium azide 0.018 mass%
· EMULGEN LS-110 (manufactured by Kao Corporation) 0.40 mass%
· Hydrochloric acid
· Water

A human biological serum sample containing albumin and γ-globulin was prepared, and diluted (dilution rate 7 times (volume basis)) with a diluent (pH 8.8) having the following composition to obtain Sample B.

To the electrophoretic liquid holding tank 3, 60 µL of the specific alkaline solution 1 was added, and the specific alkaline solution 1 was filled into the capillary flow path 1 by capillary action.

To the sample holding tank 2, 60 µL of Sample A was added.

Next, a negative electrode was brought into contact with the sample holding tank 2, a positive electrode was brought into contact with the electrophoretic liquid holding tank 3, and electrophoresis was started by applying a voltage under constant current control of 75 µA.

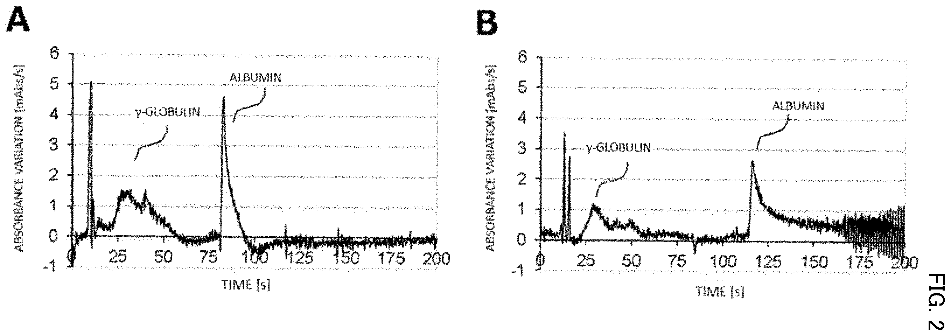

During electrophoresis, the detection unit 4 was irradiated with light of 280 nm, and the absorbance was measured to obtain an absorbance spectrum. An electropherogram was obtained by differentiating the waveform of the absorbance spectrum with respect to time. Electrophoresis was performed for 200 seconds. The obtained electropherogram is shown in Fig. 2A.

A prototype self-manufactured was used for light irradiation, absorbance measurement, and electropherogram acquisition.

An electropherogram was obtained in the same manner as described above, except that Sample A was changed to Sample B. The obtained electropherogram is shown in Fig. 2B.

As is apparent from the electropherograms shown in Figs. 2A and 2B, according to the sample analysis method of Example 1 using the specific alkaline solution containing a cationic polymer, albumin and γ-globulin could be separated within 200 seconds.

### «Example 2»

The following substances were mixed, and sodium hydroxide and water were added until the pH reached 9.8 to prepare a specific alkaline solution (capillary electrophoresis solution) 2. The content ratio of the cationic polymer with respect to the total mass of the specific alkaline solution 2 was set to 1.5 mass%.

### (Composition of Specific Alkaline Solution 2)

· Dimethylamine-ammonia-epichlorohydrin polymer 2 (cationic polymer, KHE105L manufactured by SENKA corporation, weight average molecular weight: from 100,000 to 500,000) 1.5 mass%
· Sodium azide 0.02 mass%
· EMULGEN LS-110 (manufactured by Kao Corporation) 0.1 mass%
· Sodium hydroxide aqueous solution
· Water

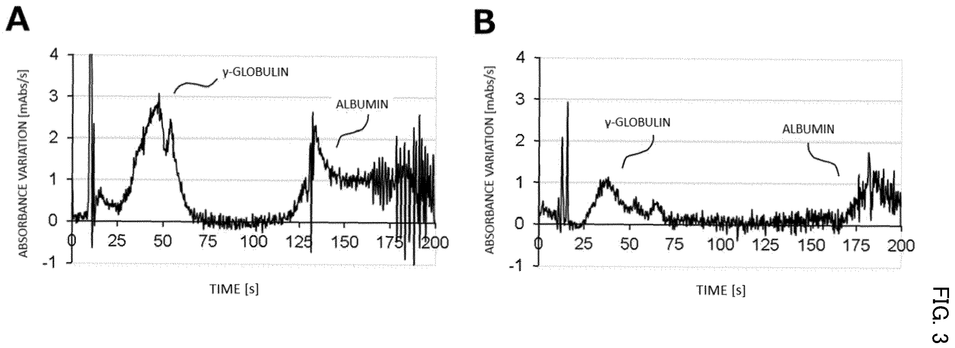

An electropherogram using Sample A and an electropherogram using Sample B were obtained in the same manner as in Example 1, except that the specific alkaline solution 1 was changed to the specific alkaline solution 2. The obtained electropherograms are shown in Figs. 3A and 3B, respectively.

As is apparent from the electropherograms shown in Figs. 3A and 3B, according to the sample analysis method of Example 2 using the specific alkaline solution containing a cationic polymer, albumin and γ-globulin could be separated within 200 seconds.

### «Example 3»

The following substances were mixed, and sodium hydroxide and water were added until the pH reached 9.8 to prepare a specific alkaline solution (capillary electrophoresis solution) 3. The content ratio of the cationic polymer with respect to the total mass of the specific alkaline solution 3 was set to 1.5 mass%.

### (Composition of Specific Alkaline Solution 3)

· Dimethylamine-epichlorohydrin polymer 1 (cationic polymer, KHE107L manufactured by SENKA corporation, weight average molecular weight: from 20,000 to 100,000) 1.5 mass%
· Sodium azide 0.02 mass%
· EMULGEN LS-110 (manufactured by Kao Corporation) 0.1 mass%
· Sodium hydroxide aqueous solution
· Water

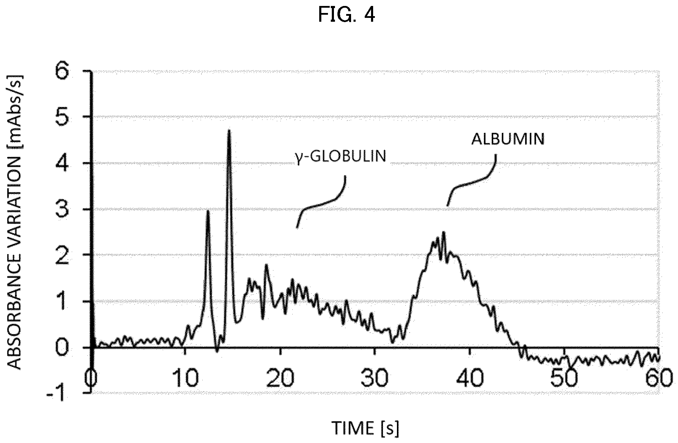

An electropherogram using Sample B was obtained in the same manner as in Example 1, except that the specific alkaline solution 1 was changed to the specific alkaline solution 3 and the electrophoresis time was changed to 60 seconds. The obtained electropherogram is shown in Fig. 4.

As is apparent from the electropherogram shown in Fig. 4, according to the sample analysis method of Example 3 using the specific alkaline solution containing a cationic polymer, albumin and γ-globulin could be separated within 60 seconds.

### «Example 4»

The following substances were mixed, and sodium hydroxide and water were added until the pH reached 9.8 to prepare a specific alkaline solution (capillary electrophoresis solution) 4. The content ratio of the cationic polymer with respect to the total mass of the specific alkaline solution 4 was set to 1.5 mass%.

### (Composition of Specific Alkaline Solution 4)

· Diallylamine salt-acrylamide polymer 1 (cationic polymer, KCA100L manufactured by SENKA corporation, weight average molecular weight: from 20,000 to 100,000) 1.5 mass%
· Sodium azide 0.02 mass%
· EMULGEN LS-110 (manufactured by Kao Corporation) 0.1 mass%
· Sodium hydroxide aqueous solution
· Water

An albumin/y-globulin mixed solution 2 having the following composition was prepared.

### (Composition of Albumin/y-Globulin Mixed Solution 2)

· 40 mg/mL albumin (manufactured by Wako, 010-27601) 4 mass/vol%
· 40 mg/mL γ-globulin (manufactured by Sigma, G4386-1G)4 mass/vol%

The albumin/y-globulin mixed solution 2 was diluted (dilution rate 7 times (volume basis)) with a diluent 2 (pH 8.8) having the following composition to obtain Sample C.

In the diluent 2, hydrochloric acid and water were added until the pH of the solution reached 8.8.

### (Composition of Diluent 2)

· Polyethyleneimine (cationic polymer, manufactured by Wako, weight average molecular weight: 70,000) 0.75 mass%
· Non-surfactant-type sulfobetaine (NDSB-201 manufactured by Tokyo Chemical Industry Co., Ltd., 3-(1-pyridino)propanesulfonic acid) 8.05 mass%
· Sucrose 7.87 mass%
· Sodium chloride 0.26 mass%
· Sodium azide 0.018 mass%
· EMULGEN LS-110 (manufactured by Kao Corporation) 0.4 mass%
· Hydrochloric acid
· Water

A human biological serum sample containing albumin and γ-globulin was prepared, and diluted (dilution rate 7 times (volume basis)) with a diluent (pH 8.8) having the following composition to obtain Sample D.

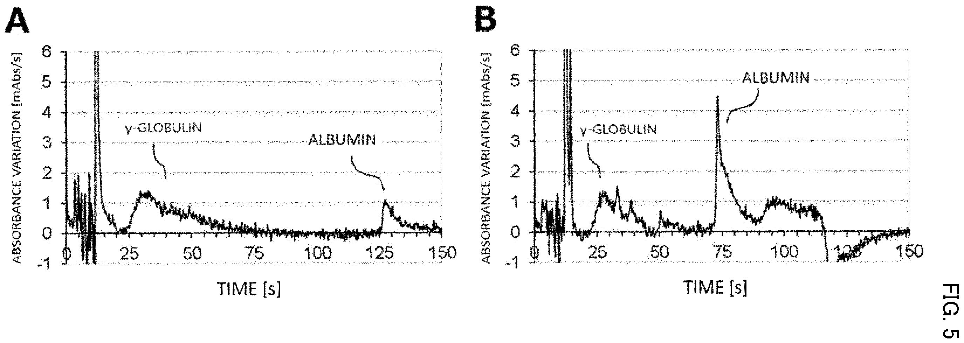

An electropherogram using Sample C or Sample D was obtained in the same manner as in Example 1, except that the specific alkaline solution 1 was changed to the specific alkaline solution 4, Sample A was changed to Sample C or Sample D, and the electrophoresis time was changed to 150 seconds. The obtained electropherograms are shown in Figs. 5A and 5B, respectively.

As is apparent from the electropherograms shown in Figs. 5A and 5B, according to the sample analysis method of Example 4 using the specific alkaline solution containing a cationic polymer, albumin and γ-globulin could be separated within 150 seconds.

### «Example 5»

The following substances were mixed, and 3-hydroxypropanesulfonic acid was added until the pH reached 9.0 to prepare a specific alkaline solution (capillary electrophoresis solution) 5. The content ratio of the cationic polymer with respect to the total mass of the specific alkaline solution 5 was set to 1.0 mass%.

### (Composition of Specific Alkaline Solution 5)

· Polyethyleneimine (cationic polymer, manufactured by Wako, weight average molecular weight: 70,000) 1.0 mass%
· Non-surfactant-type sulfobetaine (NDSB-201 manufactured by Tokyo Chemical Industry Co., Ltd., 3-(1-pyridino)propanesulfonic acid) 10.06 mass%
· Sodium azide 0.02 mass%
· 3-Hydroxypropanesulfonic acid
· Water

The albumin/y-globulin mixed solution 2 was diluted (dilution rate 7 times (volume basis)) with the specific alkaline solution 5 to obtain Sample E.

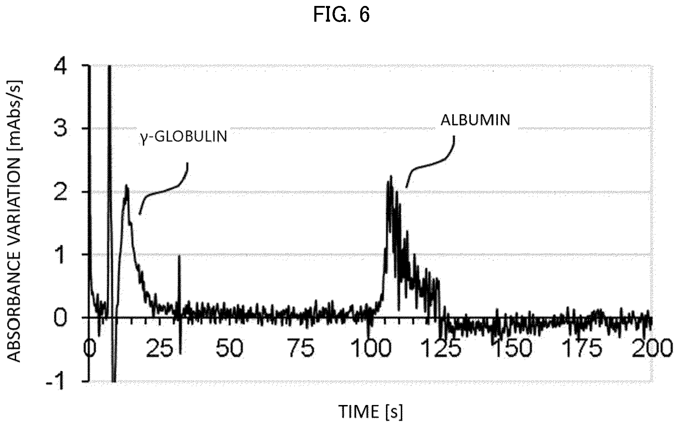

An electropherogram using Sample E was obtained in the same manner as in Example 4, except that the specific alkaline solution 4 was changed to the specific alkaline solution 5, Sample C was changed to Sample E, and the electrophoresis time was changed to 120 seconds. The obtained electropherogram is shown in Fig. 6.

As is apparent from the electropherogram shown in Fig. 6, according to the sample analysis method of Example 5 using the specific alkaline solution containing a cationic polymer, albumin and γ-globulin could be separated within 120 seconds.

### «Example 6»

The following substances were mixed, and 3-hydroxypropanesulfonic acid was added until the pH reached 9.8 to prepare a specific alkaline solution (capillary electrophoresis solution) 6. The content ratio of the cationic polymer with respect to the total mass of the specific alkaline solution 6 was set to 1.0 mass%.

### (Composition of Specific Alkaline Solution 6)

· Polyallylamine (cationic polymer, manufactured by NITTOBO MEDICAL CO., LTD., weight average molecular weight: 25,000) 1.0 mass%
· Sodium azide 0.02 mass%
· 3-Hydroxypropanesulfonic acid
· Water

The albumin/y-globulin mixed solution 2 was diluted (dilution rate 7 times (volume basis)) with a diluent 3 (pH 9.8) having the following composition to obtain Sample F.

In the diluent 3, 3-hydroxypropanesulfonic acid was added until the pH of the solution reached 9.8.

### (Composition of Diluent 3)

· Polyallylamine (cationic polymer, manufactured by NITTOBO MEDICAL CO., LTD., weight average molecular weight: 25,000) 1.0 mass%
· Non-surfactant-type sulfobetaine (NDSB-201 manufactured by Tokyo Chemical Industry Co., Ltd., 3-(1-pyridino)propanesulfonic acid) 10.06 mass%
· Sodium azide 0.02 mass%
· 3-Hydroxypropanesulfonic acid
· Water

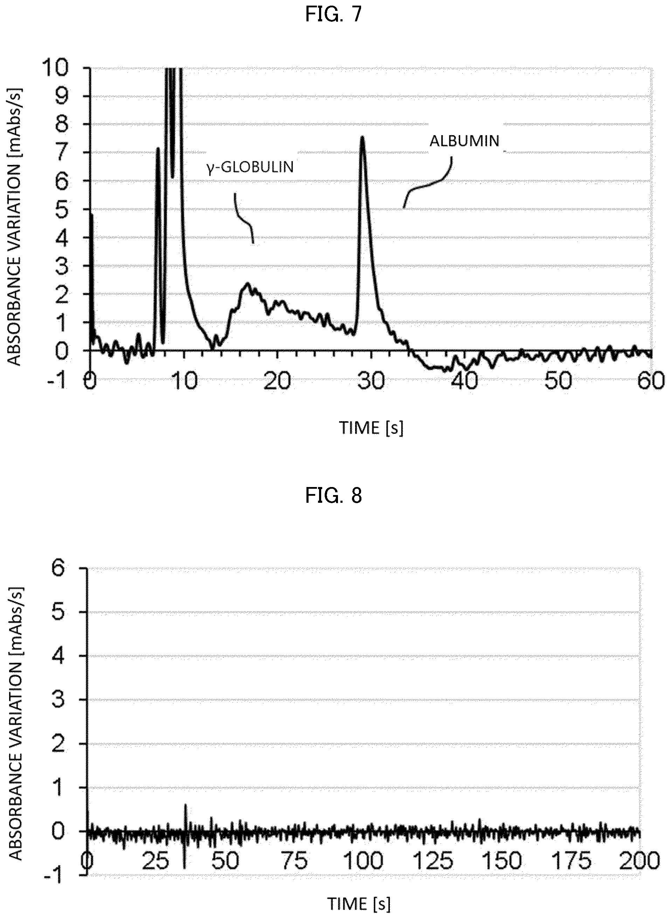

An electropherogram using Sample F was obtained in the same manner as in Example 4, except that the specific alkaline solution 4 was changed to the specific alkaline solution 6, Sample C was changed to Sample F, and the electrophoresis time was changed to 60 seconds. The obtained electropherogram is shown in Fig. 7.

As is apparent from the electropherogram shown in Fig. 7, according to the sample analysis method of Example 6 using the specific alkaline solution containing a cationic polymer, albumin and γ-globulin could be separated within 60 seconds.

### <<Comparative Example 1»

The following substances were mixed, and 3-hydroxypropanesulfonic acid was added until the pH reached 9.8 to prepare a specific alkaline solution (capillary electrophoresis solution) 10.

### (Composition of Specific Alkaline Solution 10)

· Arginine 1.74 mass%
· Sodium azide 0.02 mass%
· 3-Hydroxypropanesulfonic acid
· Water

The albumin/y-globulin mixed solution 2 was diluted (dilution rate 7 times (volume basis)) with the specific alkaline solution 10 to obtain Sample G.

An electropherogram using Sample G was obtained in the same manner as in Example 4, except that the specific alkaline solution 4 was changed to the alkaline solution 10, Sample C was changed to Sample G, and the electrophoresis time was changed to 200 seconds.

The obtained electropherogram is shown in Fig. 8.

As is apparent from the electropherograms shown in Fig. 8, according to the sample analysis method of Comparative Example 1 using the specific alkaline solution not containing a cationic polymer, albumin and γ-globulin could not be separated within 200 seconds.

### <<Comparative Example 2»

The following substances were mixed, and 3-hydroxypropanesulfonic acid was added until the pH reached 9.8 to prepare an alkaline solution (capillary electrophoresis solution) 11.

### (Composition of Specific Alkaline Solution 11)

· Arginine 1.74 mass%
· Polyethylene glycol (non-ionic polymer, manufactured by Wako) 1.0 mass%
· Sodium azide 0.02 mass%
· 3-Hydroxypropanesulfonic acid
· Water

The albumin/y-globulin mixed solution 2 was diluted (dilution rate 7 times (volume basis)) with the specific alkaline solution 11 to obtain Sample H.

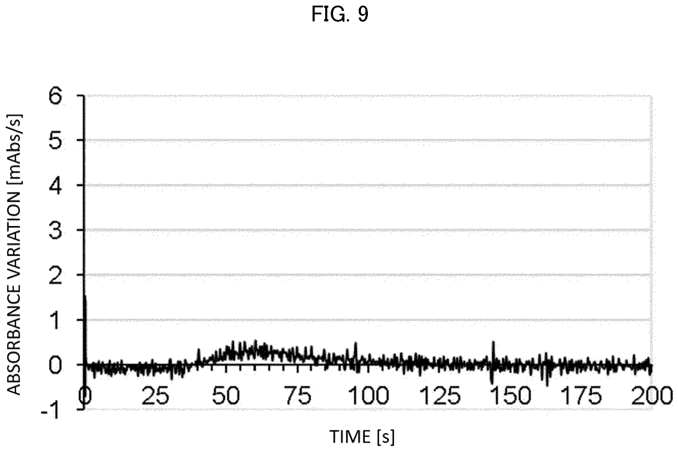

An electropherogram using Sample H was obtained in the same manner as in Example 4, except that the specific alkaline solution 4 was changed to the alkaline solution 11, Sample C was changed to Sample H, and the electrophoresis time was changed to 200 seconds. The obtained electropherogram is shown in Fig. 9.

As is apparent from the electropherogram shown in Fig. 9, according to the sample analysis method of Comparative Example 2 using the specific alkaline solution not containing a cationic polymer, albumin and γ-globulin could not be separated within 200 seconds.

## Claims

1. A sample analysis method, comprising:
a separation step of separating albumin and γ-globulin from a sample comprising albumin and γ-globulin, in an alkaline solution by capillary electrophoresis,
wherein the alkaline solution comprises a cationic polymer.

2. The sample analysis method according to claim 1, wherein a weight average molecular weight of the cationic polymer is from 10,000 to 500,000.

3. The sample analysis method according to claim 1 or 2, wherein a weight average molecular weight of the cationic polymer is from 15,000 to 150,000.

4. The sample analysis method according to claim 1 or 2, wherein the cationic polymer comprises at least one selected from the group consisting of dimethylamine-ammonia-epichlorohydrin polymer, dimethylamine-epichlorohydrin polymer, diallylamine-acrylamide polymer, polyethyleneimine, polyallylamine, polydiallylamine, allylamine-diallylamine polymer, polymethyldiallylamine, and diallyldimethylammonium chloride polymer.

5. The sample analysis method according to claim 1 or 2, wherein a content ratio of the cationic polymer with respect to a total mass of the alkaline solution is from 0.01 mass% to 10 mass%.

6. The sample analysis method according to claim 1 or 2, wherein an electrophoresis time of capillary electrophoresis in the separation is from 50 seconds to less than 250 seconds.

7. The sample analysis method according to claim 1 or 2, wherein a pH of the alkaline solution is from 8.5 to 12.0.

8. A capillary electrophoresis solution, comprising a cationic polymer,
wherein the capillary electrophoresis solution is used for separation of albumin and γ-globulin by capillary electrophoresis.

9. A sample analysis kit, comprising:
a container comprising the capillary electrophoresis solution according to claim 8; and
an electrophoresis chip comprising a sample holding tank, an electrophoretic liquid holding tank, and a capillary flow path,
wherein the sample holding tank and the electrophoretic liquid holding tank are communicated with each other via the capillary flow path.
